# EUROPÄISCHE PATENTSCHRIFT

(11) **EP 0 700 981 B1**
(45) Veröffentlichungstag und Bekanntmachung des Hinweises auf die Patenterteilung: **27.12.2000**
(21) Anmeldenummer: 95113802.3
(22) Anmeldetag: 02.09.1995
(51) Int. Cl.: C09K 19/38, C09K 19/22, C09K 19/20, C07C 69/92, C07C 235/50, C07C 251/24, C08F 20/30, G02B 5/30, G02F 1/1335

(54) **Photopolymerisierbare Flüssigkristalle**
Photopolymerisable liquid crystals
Cristaux liquides photo-polymérisables

(30) Priorität: 12.09.1994 CH 277194
(43) Veröffentlichungstag der Anmeldung: 13.03.1996
(73) Patentinhaber: Rolic AG, 6301 Zug (CH)
(72) Erfinder: Kelly, Stephen, CH-4313 Möhlin (CH); Lukàc, Teodor, CH-4123 Allschwil (CH)
(74) Vertreter: Liebetanz, Michael, Dipl.-Phys.

(56) Entgegenhaltungen:
- EP-A- 0 611 981
- WO-A-93/22397
- DE-A- 4 233 660
- US-A- 5 202 053
- POLYM. INT. (PLYIEI,09598103);93; VOL.31 (1); PP.35-40, NAGOYA UNIV.;SCH. ENG.; NAGOYA; 464; JAPAN (JP), KAWAKAMI Y ET AL 'Synthesis and thermal transition of side-chain liquid-crystalline polyoxetanes having laterally attached mesogenic group'

## Beschreibung

Die vorliegende Erfindung betrifft photochemisch oligomerisierbare oder polymerisierbare Flüssigkristalle, flüssigkristalline Gemische, die solche Verbindungen enthalten, sowie ihre Verwendung im vernetzten Zustand als optische oder elektronische Bauelemente.

Flüssigkristalle mit mindestens zwei photochemisch oligomerisierbaren oder polymerisierbaren Gruppen können auf einem Substrat oder in einer Zelle beispielsweise durch Orientierungsschichten oder in einem Feld orientiert werden. Durch Bestrahlen mit Licht einer geeigneten Wellenlänge werden diese orientierten Flüssigkristalle, versehen mit einer geeigneten Menge eines Photoinitiators, polymerisiert. Die dadurch erzeugte, vernetzte Struktur bleibt auch bei hohen Temperaturen erhalten. Solche Schichten können beispielsweise Teile von Hybridschichten sein, wie sie in den Schweizerischen Patentanmeldungen CH 2016/94 und CH 2017/94 beschrieben sind. So lassen sich optische Bauelemente, wie zum Beispiel Retarder, Wellenleiter, optische Gitter und Filter, integrierte Farbfilter, oder Zellen mit piezoelektrischen und solche mit nicht-linearen optischen (NLO) Eigenschaften, usw. herstellen. Solche optischen Bauelemente finden zum Beispiel in Projektionssystemen Einsatz.

Weitere Eigenschaften, wie beispielsweise die Doppelbrechung, die Brechungsindices, die Transparenz, usw. müssen je nach Anwendungsgebiet unterschiedlichen Anforderungen genügen. Beispielsweise sollten Netzwerke für optische Retarder eine hohe Doppelbrechung aufweisen, damit die Schichtdicke der integrierten optischen Bauelemente auf einem Minimum gehalten werden kann.

In der Mikroelektronik werden elektrisch isolierende, aber thermisch gut leitfähige Polymere eingesetzt, um bei hoher Bauteildichte die beim Betrieb entstehende Wärme schnell und effizient abzuleiten. Es besteht auch ein Bedarf an Isolierschichten mit niedriger Dielektrizitätskonstante und hoher Hitzebeständikeit. Homogen orientierte flüssigkristalline Netzwerke sind für solche Anwendungen dank ihrer kleinen thermischen Expansionskoeffizienten, hohen thermischen Leitfähigkeit und Stabilität vorteilhaft.

Neben dem generellen Interesse an photochemisch oligomerisierbaren oder polymerisierbaren Flüssigkristallen für optische und elektrische Bauelemente besteht auch in anderen Bereichen ein Bedarf für solche Verbindungen. So eignen sich solche flüssigkristallinen Materialien auch zum Beschichten von Glasfibern (Cladding) für optische Datenübertragung. Der Einsatz solcher Netzwerke erhöht den elastischen Modulus in der Längsachse der Fiber, setzt die thermischen Expansionskoeffizienten herab und vermindert Mikroverbiegungsverluste. Dies führt zu einer erhöhten mechanischen Stabilität.

Da Flüssigkristalle in der Regel als Mischungen mehrerer Komponenten zur Anwendung gelangen, ist es wichtig, dass die Komponenten untereinander gut mischbar sind. Herkömmliche photochemisch oligomerisierbare oder polymerisierbare Flüssigkristalle besitzen in der Regel einen hohen Schmelz- und Klärpunkt. Dies hat den Nachteil, dass bei der Verarbeitung, welche bei Temperaturen knapp unter dem Klärpunkt durchgeführt wird, weil bei dieser Temperatur die Viskosität im flüssigkristallinen Zustand am niedrigsten und daher günstig für eine gute Orientierbarkeit ist, vorzeitig eine spontane, thermische Polymerisation eintreten kann. Diese spontane Polymerisation führt zu Domänenbildung, wodurch die optischen und thermischen Eigenschaften in den erzeugten, vernetzten Schichten deutlich beeinträchtig werden. Der Schmelzpunkt kann durch die Herstellung komplizierter Mischungen mit mehreren Komponenten herabgesetzt werden, was zwar eine Verarbeitung bei niedrigeren Temperaturen erlaubt, aber die Gefahr einer Kristallisation der herkömmlichen polymerisierbaren Flüssigkristalle mit sich bringt.

Es stellte sich somit die Aufgabe, photochemisch oligomerisierbare oder polymerisierbare Verbindungen herzustellen, welche den obengenannten Anforderungen genügen. Sie sollten niedrigere Schmelz- und Klärpunkte besitzen, damit sie bei Temperaturen oberhalb Raumtemperatur im flüssigkristallinen Zustand und auch in Lösung sehr gut verarbeitbar sind. Weiters sollten sie möglichst domänenfrei orientierbar und strukturierbar sein. Ausserdem sollten sie eine ausgezeichnete thermische Stabilität und Langzeitstabilität im vernetzten Zustand aufweisen.

Die vorliegende Erfindung stellt nun Verbindungen, die in hervorragender Weise als Einzelkomponenten oder als Komponenten solcher Flüssigkristall-Mischungen geeignet sind, zur Verfügung. Gegenstand der vorliegenden Erfindung sind Verbindungen der allgemeinen Formel 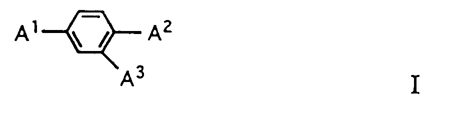 in welcher
- A¹, A², A³: unabhängig voneinander für je einen photochemisch oligomerisierbaren oder polymerisierbaren mesogenen Rest der Formel II stehen.

Mesogene Reste sind dem Fachmann bekannt und sind beispielsweise in "Flüssigkristalle in Tabellen, Vol. II, Deutscher Verlag für Grundstoffindustrie, Leipzig, 1984" beschrieben. Damit diese bekannten mesogenen Reste für obige Zwecke geeignet sind, werden sie endständig mit einem polymerisierbaren Rest versehen.

Ehe Verbindungen der allgemeinen Formel I sind in üblichen Lösungsmitteln wie beispielsweise in Anisol, N' N-Dimethylacetamid oder N-Methylpyrrolidon gut löslich und zeichnen sich durch eine verhältnismässig niedrige Viskosität aus. Sie können daher problemlos auf eine geeignete Oberfläche aufgetragen werden. Dies geschieht in der Regel durch Aufschleudern ("Spin-Coating"). Da die erfindungsgemässen Verbindungen zudem eine flüssigkristalline Phase aufweisen, können sie vor dem Vernetzen auf einer Orientierungsschicht oder durch Anlegen eines elektrischen oder magnetischen Feldes ausgerichtet werden.

Erfindungsgemäss sind die photochemisch oligomerisierbaren oder polymerisierbaren mesogenen Reste A¹, A² und A³ unabhängig voneinander Reste der Formel 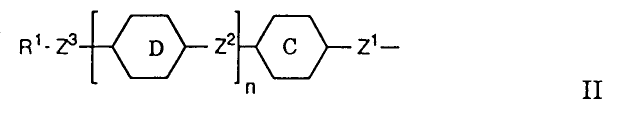 in welcher
- C und D: unabhängig voneinander gegebenenfalls mit Nitro, Acetyl, Halogen, Methyl und/oder Cyano substituiertes 1,4-Phenylen, Pyridin-2,5-diyl, Pyrimidin-2,5-diyl, trans-1,4-Cyclohexylen oder trans-1,3-Dioxan-2,5-diyl;
- Z¹: eine Einfachbindung, -CH₂CH₂-, -CH₂O-, -COO-, -OOC-, -(CH₂)₄- oder -(CH₂)₃O- ;
- Z²: eine Einfachbindung, -CH₂CH₂-, -CH₂O-, -OCH₂-, -COO-, -OOC-, -(CH₂)₄-, -O(CH₂)₃- oder -(CH₂)₃O-;
- Z³: -(CY₂)ₘ-, -(CY₂)ₘO-, -(CY₂)ₘCOO-, -(CY₂)ₘOOC, -(Si[(CH₃)₂])ₚ-, -OCH₂(Si[(CH₃)₂]O)ₚSi[(CH₃)₂]CH₂O- oder -NHCH₂(Si[(CH₃)₂]O)ₚSi[(CH₃)₂]CH₂NH-;
- Y: Wasserstoff oder Fluor;
- m: eine ganze Zahl von 1 bis 16;
- p: eine ganze Zahl von 1 bis 16
- n: 0 oder 1; und
- R¹: eine polymerisierbare Gruppe wie Acrylat, Allylether, Allylester, Methacrylat, 2-Chlor-acrylat, 2-Phenylacrylat, Acryloylphenylen, Acrylamid, Methacrylamid, 2-Phenylacrylamid, Epoxy, Itaconsäureester, Vinylether, Vinylester, Styrol-Derivate, Maleinsäure-Derivate, Fumarsäure-Derivate oder eine dimerisierbare Gruppe, wie beispielsweise Chalcon oder ein gegebenenfalls mit Methyl, Methoxy, Cyano und/oder Halogen substituiertes Zimtsäurederivat, bedeuten.

Der Ausdruck "gegebenenfalls mit Nitro, Acetyl, Halogen, Methyl und/oder Cyano substituiertes 1,4-Phenylen" bedeutet im Rahmen der vorliegenden Erfindung 1,4-Phenylen, 2-Nitro-1,4-phenylen, 3-Nitro-1,4-phenylen, 2-Acetyl-1,4-phenylen, 3-Acetyl-1,4-phenylen, 2-Fluor-1,4-phenylen, 3-Fluor-1,4-phenylen, 2,3-Difluor-1,4-phenylen, 2-Chlor-1,4-phenylen, 3-Chlor-1,4-phenylen, 2,3-Dichlor-1,4-phenylen, 2-Cyano-1,4-phenylen, 3-Cyano-1,4-phenylen, 2,3-Dicyano-1,4-phenylen, 2-Methyl-1,4-phenylen, 3-Methyl-1,4-phenylen und dergleichen.

Verbindungen der Formel I, worin zwei der mesogenen Reste A¹, A² und A³ die gleiche Bedeutung haben, werden bevorzugt. Es sind dies Verbindungen der Formeln 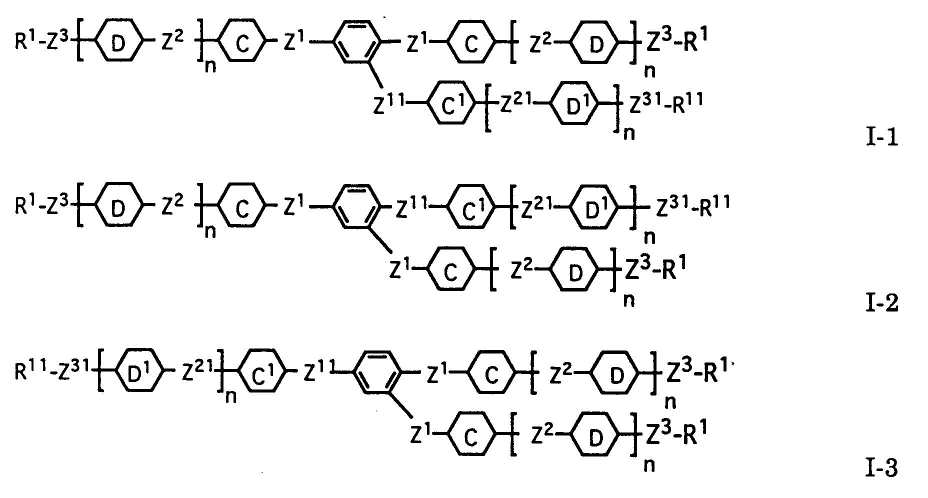 worin
- C, C¹, D und D¹: unabhängig voneinander gegebenenfalls mit Nitro, Acetyl, Halogen, Methyl und/oder Cyano substituiertes 1,4-Phenylen, Pyridin-2,5-diyl, Pyrimidin-2,5-diyl, trans-1,4-Cyclohexylen oder trans-1,3-Dioxan-2,5-diyl; Z¹ und Z¹¹ unabhängig voneinander eine Einfachbindung, -CH₂CH₂-, -CH₂O-, -COO-, -OOC-, -(CH₂)₄- oder -(CH₂)₃O- ;
- Z² und Z²¹: unabhängig voneinander eine Einfachbindung, -CH₂CH₂-, -CH₂O-, -OCH₂-, -COO-, -OOC-, -(CH₂)₄-, -O(CH₂)₃- oder -(CH₂)₃O-;
- Z³ und Z³¹: unabhängig voneinander -(CY₂)ₘ-, -(CY₂)ₘO-, -(CY₂)ₘCOO-, -(CY₂)ₘOOC, -(Si[(CH₃)₂]O)ₚ-, -OCH₂(Si[(CH₃)₂]O)ₚSi[(CH₃)₂]CH₂O- oder -NHCH₂(Si[(CH₃)₂]O)ₚSi[(CH₃)₂]CH₂NH-;
- Y: Wasserstoff oder Fluor;
- m: eine ganze Zahl von 1 bis 16;
- p: eine ganze Zahl von 1 bis 16;
- n: 0 oder 1; und
- R¹ und R¹¹: unabhängig voneinander eine polymerisierbare Gruppe wie Acrylat, Allylether, Allylester, Methacrylat, 2-Chlor-acrylat, 2-Phenylacrylat, Acryloylphenylen, Acrylamid, Methacrylamid, 2-Phenylacrylamid, Epoxy, Itaconsäureester, Vinylether, Vinylester, Styrol-Derivate, Maleinsäure-Derivate, Fumarsäure-Derivate oder eine dimerisierbare Gruppe, wie beispielsweise Chalcon oder ein gegebenenfalls mit Methyl, Methoxy, Cyano und/oder Halogen substituiertes Zimtsäurederivat, bedeuten.

Ganz besonders bevorzugte Verbindungen der Formel I sind diejenigen, worin die mesogenen Reste A¹ , A² und A³ gleich sind, dh. Verbindungen der Formel 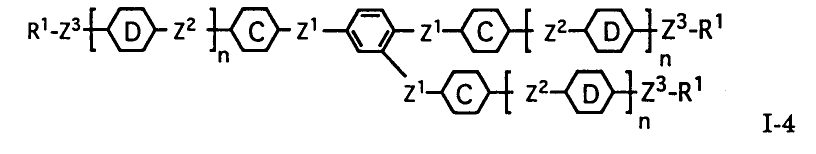 worin die Symbole obengenannte Bedeutungen haben.

Der Mesophasentyp der erfindungsgemässen Verbindungen der Formeln I-1 bis I-4 kann durch Variation der Ringe C bzw. C¹ und D bzw. D¹ beeinflusst werden. So haben heterocyclische Ringe eher die Tendenz smektische Phasen zu erzeugen, wahrend Phenylen- und Cyclohexylen-Ringe nematische Tendenzen fördern. Vorzugsweise bedeuten die Ringe C bzw. C¹ und D bzw. D¹ unabhängig voneinander 1,4-Phenylen, 2-Fluor-1,4-phenylen, 3-Fluor-1,4-phenylen, Pyridin-2,5-diyl oder Pyrimidin-2,5-diyl, insbesondere 1,4-Phenylen, 2-Fluor-1,4-phenylen oder 3-Fluor-1,4-phenylen.

Besonders bevorzugte Verbindungen der Formeln I-1 bis I-4 sind Verbindungen, worin Z¹ bzw. Z¹¹-CH₂O-, -COO- oder -OOC-; Z² bzw. Z²¹ eine Einfachbindung, -CH₂CH₂, -CH₂O-, -OCH₂-, -COO- oder -OOC-; und Y Wasserstoff bedeuten.

In besonders bevorzugten Verbindungen der Formeln I-1 bis I-4 bedeuten die Reste R¹ bzw. R¹¹ Acrylat, Methacrylat, 2-Chloracrylat, 2-Phenylacrylat, Acryloylphenylen, Acrylamid, Methacrylamid, 2-Phenylacrylamid, Epoxy, Vinylether, Vinylester, Styrol-Derivate, Maleinsäure-Derivate, oder Fumarsäure-Derivate und dergleichen; ganz besonders bevorzugt sind Acrylat, Methacrylat, Vinylether und Epoxy.

Vorzugsweise bedeuten die mesogenen Reste A¹, A² und A³ einen Rest der Formel II, worin n = 0 ist, wie beispielsweise 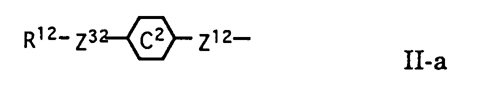 worin
- R¹²: Acrylat, Methacrylat oder Epoxy;
- Z³²: -(CH₂)ₜ-, -(CH₂)ₜO-, -(CH₂)ₜCOO- oder -(CH₂)ₜOOC-;
- t: eine ganze Zahl von 3 bis 12;
- C²: 1,4-Phenylen, 2-Fluor-1,4-phenylen, 3-Fluor-1,4-phenylen; und
- Z¹²: -CH₂O-, -COO- oder -OOC- bedeutet.

Ganz besonders bevorzugt sind die Verbindungen der Formel 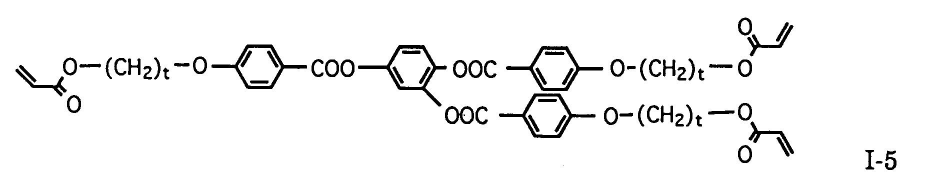 worin t eine ganze Zahl von 3 bis 12 bedeutet.

Die erfindungsgemässen Verbindungen der allgemeinen Formel I, I-1, I-2 und I-3, worin die mesogenen Reste A¹, A² und A³ voneinander verschieden sind, sind synthetisch sehr einfach zugänglich und können auf an sich bekannte Weise, beispielsweise analog zu der in Schemata 1 bis 3 sowie in den Beispielen illustrierten Methode, hergestellt werden.

Die mesogenen Reste der Formel II und II-a sind bekannt oder Analoge bekannter Strukturen und können in an sich bekannter Weise hergestellt werden und anschliessend mit dem Aromaten verknüpft werden.

Eine kleine Menge BHT (2, 6-Di-tert.-butyl-4-methyl-phenol = "Butylhydroxytoluol") wird jeder Stufe beigemischt, um unerwünschtes thermisches Vernetzen zu unterbinden.

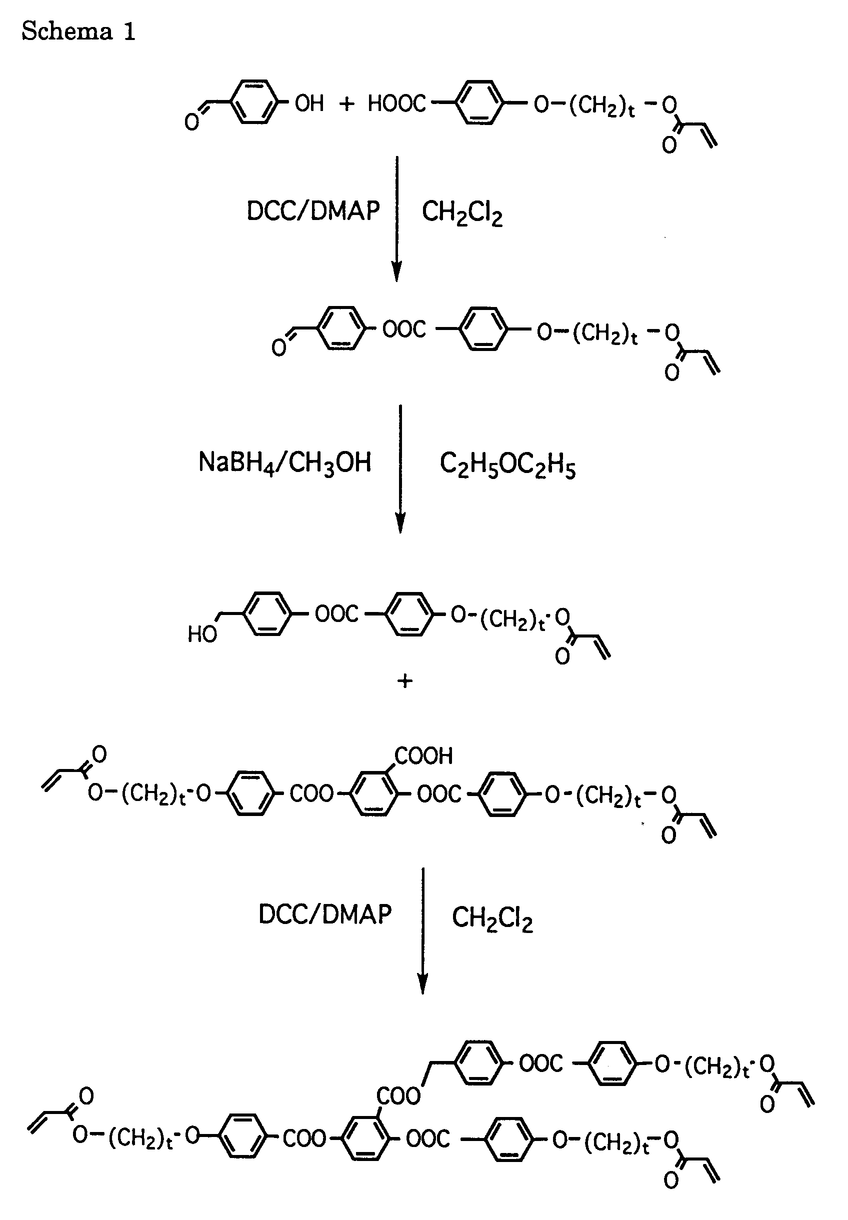

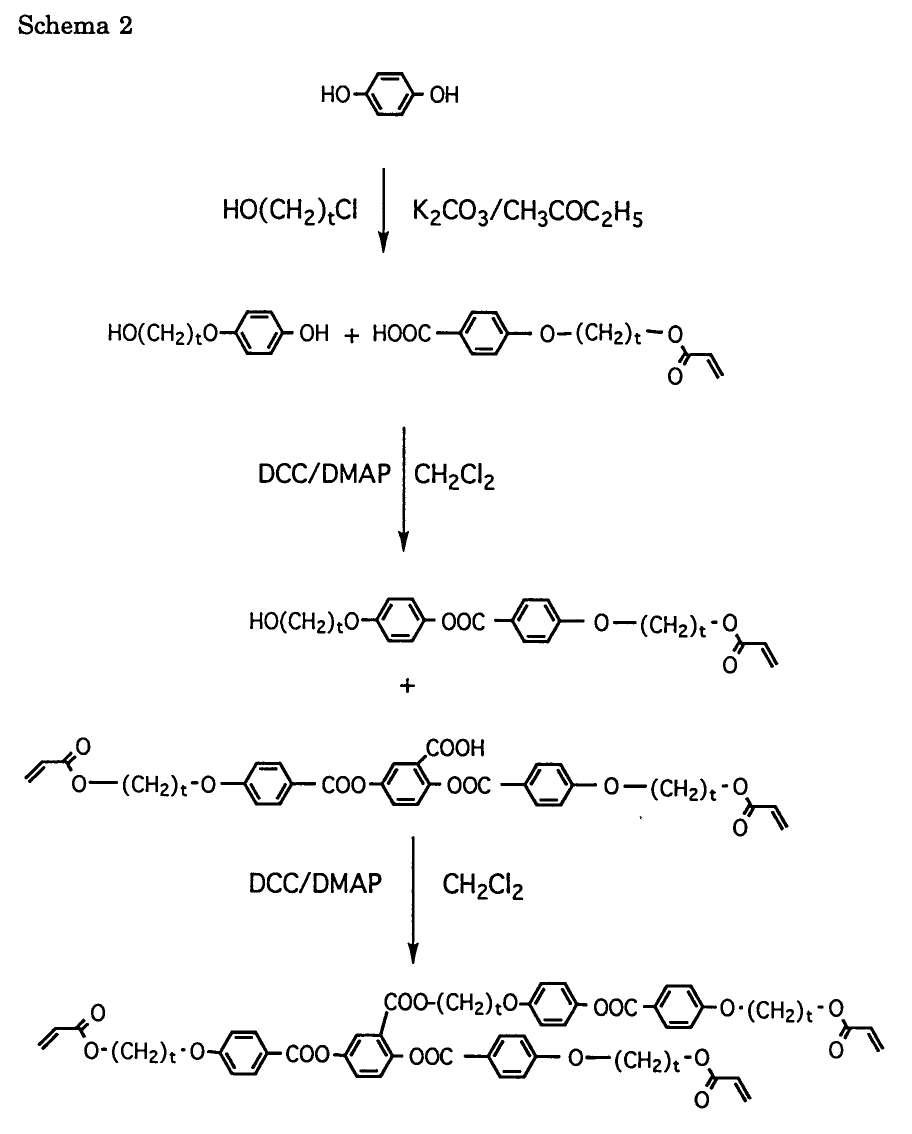

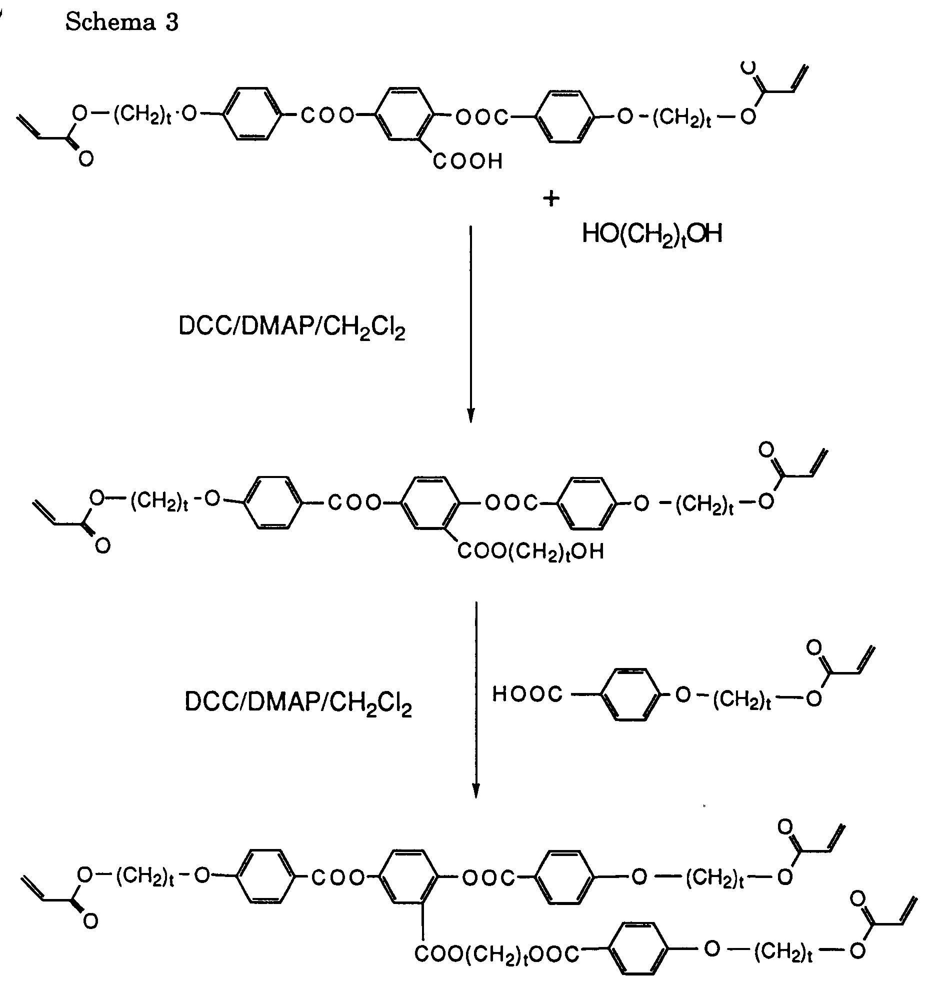

Verbindungen der Formeln I-4 und I-5, worin die mesogenen Reste A¹, A² und A³ gleich sind, können beispielsweise in einfacher und an sich bekannter Weise aus 1,2,4-Trihydroxyphenol und den entsprechenden Säure-Derivaten der Formel II wie 4-[n-Acryloyloxyalkyloxy)]benzoesäuren gemäss Schema 4 hergestellt werden. Die Veresterung kann beispielsweise in Gegenwart von N,N'-Dicyclohexylcarbodiimid und 4-(Dimethylamino)pyridin in Dichlormethan oder einem anderen geeigneten Lösungsmittel wie z.B. Chloroform erfolgen. Die Ausgangsmaterialien sind bekannt und z.T. im Handel erhältlich. 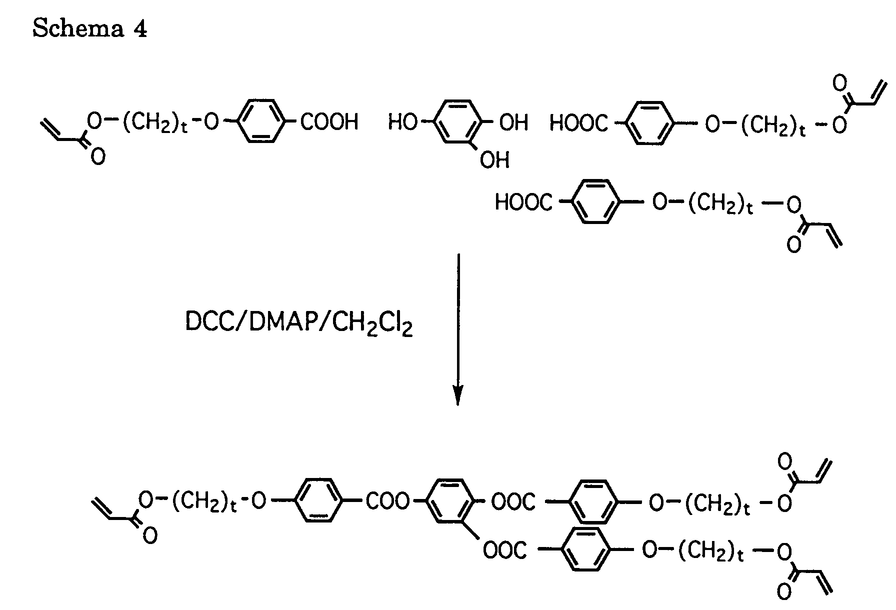 Die Verbindungen der Formeln I können als reine Verbindungen oder in Form von Gemischen untereinander und/oder mit anderen Flüssigkristallkomponenten verwendet werden.

Die erfindungsgemässen flüssigkristallinen Gemische enthalten mindestens 2 Komponenten, wovon mindestens eine Komponente eine Verbindung der Formel I ist. Eine zweite und gegebenenfalls weitere Komponenten können weitere Verbindungen der Formel I oder auch andere, bekannte flüssigkristalline Verbindungen mit oder ohne photovernetzbare Gruppen sein. Es können auch eine oder mehrere chirale Komponenten im Gemisch enthalten sein.

Aufgrund der guten Löslichkeit der Verbindungen der Formel I und aufgrund ihrer guten Mischbarkeit untereinander kann der Anteil an verschiedenen Verbindungen der Formel I in den erfindungsgemässen Gemischen hoch sein und bis 100 Gew.-% betragen.

Vorzugsweise enthalten die erfindungsgemässen Gemische neben einer oder mehreren Verbindungen der Formel I eine oder mehrere Verbindungen aus der Gruppe der Verbindungen der allgemeinen Formeln 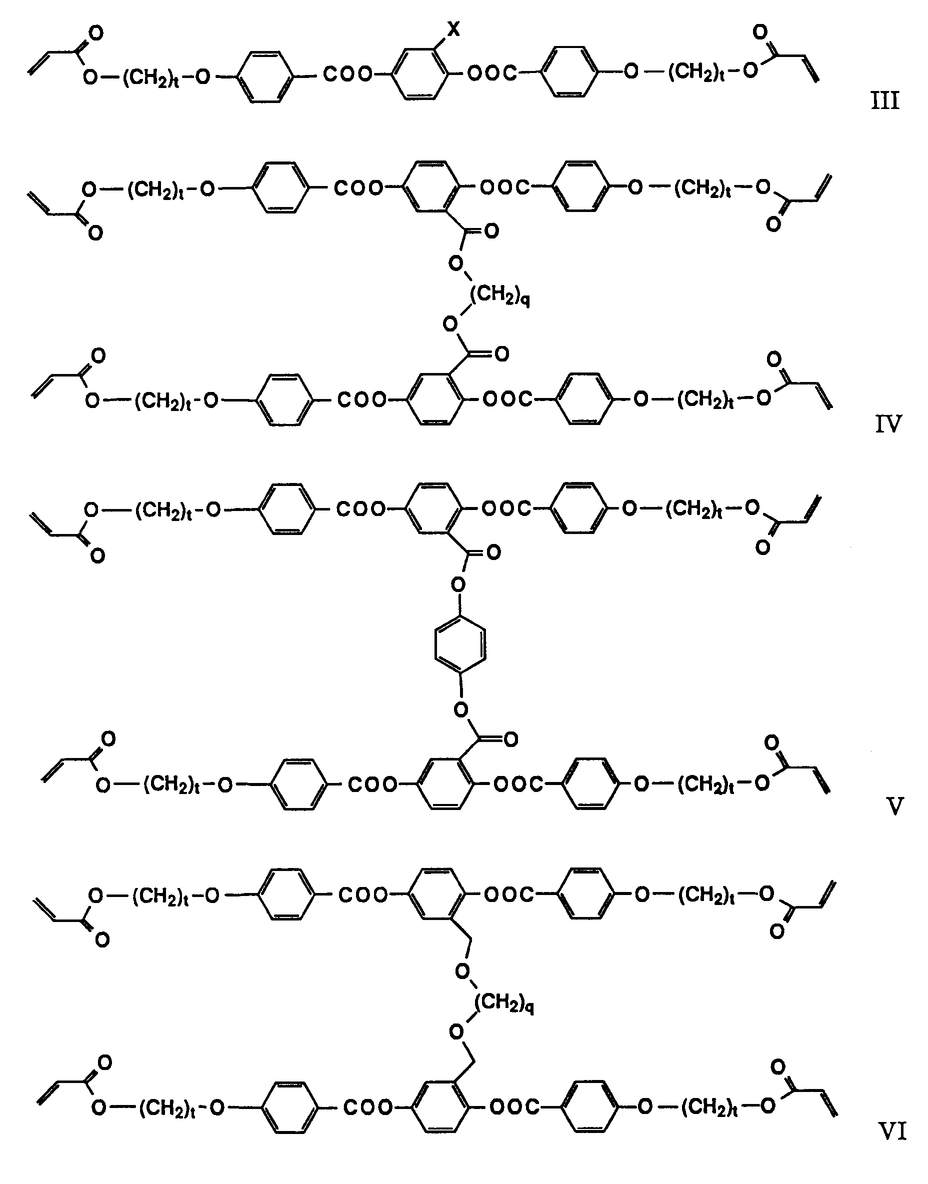 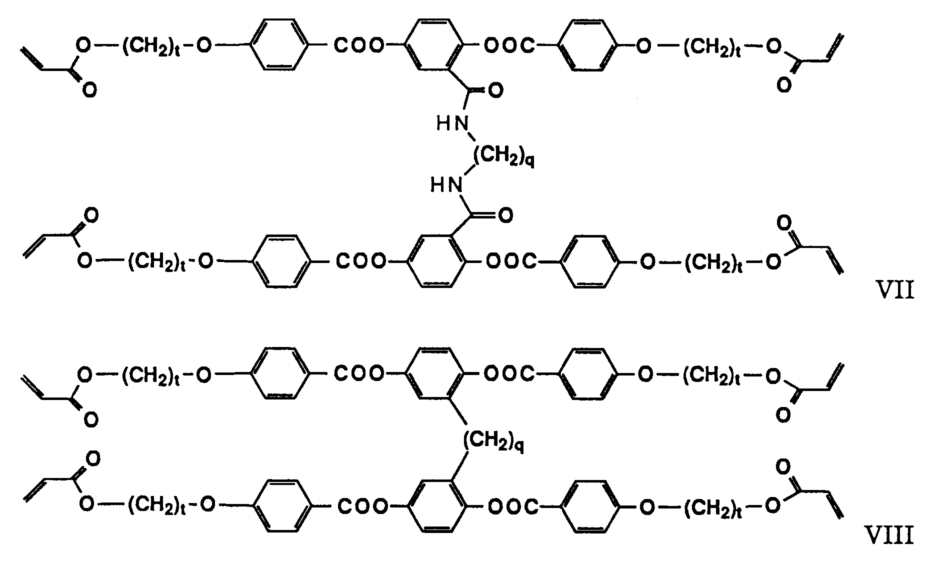 worin
- t: eine ganze Zahl von 3 bis 12;
- q: eine ganze Zahl von 2 bis 12; und
- X: Wasserstoff, Fluor, Chlor oder niederes Alkyl wie Methyl, Ethyl, Propyl oder Butyl bedeuten.

Die Herstellung der Verbindungen der Formel I sowie flüssigkristalliner Gemische enthaltend diese Verbindungen werden durch die folgenden Beispiele weiter veranschaulicht. C bedeutet eine kristalline, S eine smektische, N eine nematische und I die isotrope Phase.

### Beispiel 1

Zu einer Lösung von 4,7 g 4-[6-Acryloyloxyhexyloxy)]benzoesäure, 0,5 g Trihydroxybenzol und 0,2 g 4-Dimethylaminopyridin in 25 ml Dichlormethan wurde unter Rühren bei Raumtemperatur 5,0 g N, N'-Dicyclohexyldicarbodiimid gegeben. Das Reaktionsgemisch wurde über Nacht bei Raumtemperatur gerührt, auf 100 ml Wasser gegossen und dann dreimal mit je 50 ml Dichlormethan extrahiert. Die vereinigten organischen Phasen wurden zweimal mit je 100 ml Wasser gewaschen, über Magnesiumsulfat getrocknet, filtriert und das Filtrat anschliessend eingeengt. Chromatographische Reinigung des Rückstandes an Kieselgel mit Hexan/Ethylacetat (Vol. 3:1) und zweimaliges Umkristallisieren der gemäss Dünnschichtchromatographie reinen Fraktionen aus Ethylalkohol ergab 2,0 g 1,2,4-Tri(4-[6-acryloyl-oxyhexyloxy]-phenylcarbonyloxy)-benzol; Smp. (C-I) 44^{o}C, Klp. (N-I) 33^{o}C.

In analoger Weise wurden folgende Verbindungen hergestellt:
1,2,4-Tri(4-[3-acryloyloxypropyloxy]phenylcarbonyloxy)benzol;
1,2,4-Tri(4-[4-acryloyloxybutyloxy]phenylcarbonyloxy)benzol; Smp. (C-I) 46°C, Klp. (N-I) (-8°C).
1,2,4-Tri(4-[5-acryloyloxypentyloxy]phenylcarbonyloxy)benzol; Smp. (C-I) 39°C, Klp. (N-I) 33°C.
1,2,4-Tri(4-[7-acryloyloxyheptyloxy]phenylcarbonyloxy)benzol; Smp. (C-N) 31°C, Klp. (N-I) 44°C.
1,2,4-Tri(4-[8-acryloyloxyoctyloxy]phenylcarbonyloxy)benzol; Smp. (C-I) 56°C, Klp. (N-I) 42°C.
1,2,4-Tri(4-[9-acryloyloxynonyloxy]phenylcarbonyloxy)benzol; Smp. (C-N) 41°C, Klp. (N-I) 51°C.
1,2,4-Tri(4-[10-acryloyloxydecyloxy]phenylcarbonyloxy)benzol; Smp. (C-I) 55°C, Klp. (N-I) 46°C.
1,2,4-Tri(4-[11-acryloyloxyundecyloxy]phenylcarbonyloxy)benzol; Smp. (C-N) 44°C, Klp. (N-I) 53°C.

### Beispiel 2

Zu einer Lösung von 0,6 g 2,5-bis(4-[6-Acryloyloxyhexyloxy]phenylcarbonyloxy)-benzoesäure, 0,4 g [4-(4-[6-Acryloyloxy-hexyloxy)]phenylcarbonyloxy)phenyl]methanol und 0,04 g 4-Dimethylamino-pyridin in 20 ml Dichlormethan wird unter Rühren bei Raumtemperatur 0,2 g N, N'-Dicyclohexyldicarbodiimid gegeben. Das Reaktionsgemisch wird über Nacht bei Raumtemperatur gerührt, auf 100 ml Wasser gegossen und dann dreimal mit je 50 ml Dichlormethan extrahiert. Die vereinigten organischen Phasen werden zweimal mit je 100 ml Wasser gewaschen, über Magnesiumsulfat getrocknet, filtriert und das Filtrat anschliessend eingeengt. Chromatographische Reinigung des Rückstandes an Kieselgel mit Hexan/Aethylacetat (Vol. 1:1) und zweimaliges Umkristallisieren der gemäss Dünnschichtchromatographie reinen Fraktionen aus Aceton ergibt 0,4 g [4-(4-[6-Acryloyloxyhexyloxy)]-phenylcarbonyloxy)-phenyl]-methyl 2,5-bis(4-[6-acryloyloxyhexyloxy)]-phenylcarbonyloxy)-benzoesäureester.

Der als Ausgangsmaterial verwendete [4-(4-[6-Acryloyloxy-hexyloxy]phenylcarbonyloxy)-phenyl]-methanol wird wie folgt hergestellt:
(a) Ein Gemisch von 0,13 g Natriumborhydrid und 15 ml Wasser wird tropfenweise bei 0^{o}C mit einer Lösung von 0,8 g 4-(4-[6-Acryloyloxyhexyloxy]phenylcarbonyloxy)benzaldehyd in 100 ml Dioxan versetzt. Das Reaktionsgemisch wird weitere 60 Minuten bei 0^{o}C und dann 10 Minuten bei Raumtemperatur gerührt, auf 100 ml Dichlormethan gegossen und zweimal mit je 100 ml Wasser gewaschen. Die vereinigten wässrigen Phasen werden zweimal mit je 50 ml Dichlormethan extrahiert. Die vereinigten organischen Phasen werden dann zweimal mit je 100 ml Wasser gewaschen, über Magnesiumsulfat getrocknet, die Suspension filtriert und das Filtrat eingeengt. Der Rückstand wird ohne weitere Reinigung in die nächste Stufe eingesetzt.
(b) Zu einer Lösung von 1,0 g 4-[6-Acryloyloxyhexyloxy]benzoesäure, 0,3 g 4-Hydroxybenzaldehyd und 0,04 g 4-Dimethylaminopyridin in 20 ml Dichlormethan wird unter Rühren bei Raumtemperatur 0,6 g N, N'-Dicyclodicyclohexyldicarbodiimid gegeben. Das Reaktionsgemisch wird über Nacht bei Raumtemperatur gerührt, auf 100 ml Wasser gegossen und dann dreimal mit je 50 ml Dichlormethan extrahiert. Die vereinigten organischen Phasen werden zweimal mit je 100 ml Wasser gewaschen, über Magnesiumsulfat getrocknet, filtriert und das Filtrat anschliessend eingeengt. Chromatographische Reinigung des Rückstandes an Kieselgel mit Hexan/Aethylacetat (Vol. 1:1) und zweimaliges Umkristallisieren der gemäss Dünnschichtchromatographie reinen Fraktionen aus Aethanol ergibt 0,9 g 4-(4-[6-Acryloyloxyhexyloxy]phenylcarbonyloxy)benzaldehyd.
In analoger Weise können folgende Verbindungen hergestellt werden:
[4-(4-[6-Acryloyloxyhexyloxy)]phenylcarbonyloxy)phenyl]methyl 2,5-bis(4-[7-acryloyloxyheptyloxy]phenylcarbonyloxy)benzoesäureester;
[4-(4-[6-Acryloyloxyhexyloxy)]phenylcarbonyloxy)phenyl]methyl 2,5-bis(4-[8-acryloyloxyoctyloxy]phenylcarbonyloxy)benzoesäureester;
[4-(4-[6-Acryloyloxyhexyloxy)]phenylcarbonyloxy)phenyl]methyl 2,5-bis(4-[9-acryloyloxynonyloxy]phenylcarbonyloxy)benzoesäureester;
[4-(4-[6-Acryloyloxyhexyloxy)]phenylcarbonyloxy)phenyl]methyl 2,5-bis(4-[10-acryloyloxydecyloxy]phenylcarbonyloxy)benzoesäureester;
[4-(4-[6-Acryloyloxyhexyloxy)]phenylcarbonyloxy)phenyl]methyl 2,5-bis(4-[11-acryloyloxyundecyloxy]phenylcarbonyloxy)benzoesäureester;
[4-(4-[6-Acryloyloxyhexyloxy)]phenylcarbonyloxy)phenyl]methyl 2,5-bis(4-[12-acryloyloxydodecyloxy]phenylcarbonyloxy)benzoesäureester;
[4-(4-[3-Acryloyloxypropyloxy)]phenylcarbonyloxy)phenyl]methyl 2,5-bis(4-[7-acryloyloxyheptyloxy]phenylcarbonyloxy)benzoesäureester;
[4-(4-[4-Acryloyloxybutyloxy)]phenylcarbonyloxy)phenyl]methyl 2,5-bis(4-[8-acryloyloxyoctyloxy]phenylcarbonyloxy)benzoesäureester;
[4-(4-[5-Acryloyloxypentyloxy)]phenylcarbonyloxy)phenyl]methyl 2,5-bis(4-[9-acryloyloxynonyloxy]phenylcarbonyloxy)benzoesäureester;
[4-(4-[7-Acryloyloxyheptyloxy)]phenylcarbonyloxy)phenyl]methyl 2,5-bis(4-[10-acryloyloxydecyloxy]phenylcarbonyloxy)benzoesäureester;
[4-(4-[8-Acryloyloxyoctyloxy)]phenylcarbonyloxy)phenyl]methyl 2,5-bis(4-[12-acryloyloxydodecyloxy]phenylcarbonyloxy)benzoesäureester;
[4-(4-[9-Acryloyloxynonyloxy)]phenylcarbonyloxy)phenyl]methyl 2,5-bis(4-[11-acryloyloxyundecyloxy]phenylcarbonyloxy)benzoesäureester;
[4-(4-[10-Acryloyloxydecyloxy)]phenylcarbonyloxy)phenyl]methyl 2,5-bis(4-[11-acryloyloxyundecyloxy]phenylcarbonyloxy)benzoesäureester;
[4-(4-[11-Acryloyloxyundecyloxy)]phenylcarbonyloxy)phenyl]methyl 2,5-bis(4-[11-acryloyloxyundecyloxy]phenylcarbonyloxy)benzoesäureester;
[4-(4-[12-Acryloyloxydodecyloxy)]phenylcarbonyloxy)phenyl]methyl 2,5-bis(4-[12-acryloyloxydodecyloxy]phenylcarbonyloxy)benzoesäureester.

### Beispiel 3

0,6 g 2,5-bis(4-[6-Acryloyloxyhexyloxy]-phenylcarbonyloxy)benzoesäure, 0,4 g [4-(4-[6-Acryloyloxyhexyloxy]-phenylcarbonyloxy]phenoxy)ethanol und 0,04 g 4-Dimethylaminopyridin, 0,2 g N, N'-Dicyclodicyclohexydicarbodiimid und 20 ml Dichlormethan werden in analoger Weise zu Beispiel 1 zu [4-(4-[6-Acryloyloxyhexyloxy]phenylcarbonyloxy)-phenoxy]ethyl 2,5-bis(4-[6-acryloyloxyhexyloxy)]phenylcarbonyloxy)benzoesäureester umgesetzt.

Der als Ausgangsmatrial verwendete [4-(4-[6-Acryloyloxyhexyloxy]phenylcarbonyloxy)phenoxy]ethanol wird wie folgt hergestellt:
(a) 0,6 g 4-[6-Acryloyloxyhexyloxy)]benzoesäure, 0,2 g (4-Hydroxyphenoxy)ethanol und 0,04 g 4-Dimethylaminopyridin, 0,4 g N, N'-Dicyclodicyclohexyldicarbodiimid und 50 ml Dichlormethan werden in analoger Weise zu Beispiel 1 zu 0,4 g (4-[6-Acryloyloxyhexyloxy)]phenylcarbonyloxy]-phenoxy)ethanol umgesetzt.
In analoger Weise können folgende Verbindungen hergestellt werden:
[4-(4-[6-Acryloyloxyhexyloxy]phenylcarbonyloxy)phenoxy]ethyl 2,5-bis(4-[3-acryloyloxypropyloxy)]phenylcarbonyloxy)benzoesäureester;
[4-(4-[6-Acryloyloxyhexyloxy]phenylcarbonyloxy)phenoxy]ethyl 2,5-bis(4-[4-acyloyloxybutyloxy)]phenylcarbonyloxy)benzoesäureester;
[4-(4-[6-Acryloyloxyhexyloxy]phenylcarbonyloxy)phenoxy]ethyl 2,5-bis(4-[5-acryloyloxypentyloxy)]phenylcarbonyloxy)benzoesäureester;
[4-(4-[6-Acryloyloxyhexyloxy]phenylcarbonyloxy)phenoxy]ethyl 2,5-bis(4-[7-acryloyloxyheptyloxy)]phenylcarbonyloxy)benzoesäureester;
[4-(4-[6-Acryloyloxyhexyloxy]phenylcarbonyloxy)phenoxy]ethyl 2,5-bis(4-[8-acryloyloxyoctyloxy)]phenylcarbonyloxy)benzoesäureester;
[4-(4-[6-Acryloyloxyhexyloxy]phenylcarbonyloxy)phenoxy]ethyl 2,5-bis(4-[9-acryloyloxynonyloxy)]phenylcarbonyloxy)benzoesäureester;
[4-(4-[6-Acryloyloxyhexyloxy]phenylcarbonyloxy)phenoxy]ethyl 2,5-bis(4-[10-acryloyloxydecyloxy)]phenylcarbonyloxy)benzoesäureester;
[4-(4-[6-Acryloyloxyhexyloxy]phenylcarbonyloxy)phenoxy]ethyl 2,5-bis(4-[11-acryloyloxyundecyloxy)]phenylcarbonyloxy)benzoesäureester;
[4-(4-[6-Acryloyloxyhexyloxy]phenylcarbonyloxy)phenoxy]ethyl 2,5-bis(4-[12-acryloyloxydodecyloxy)]phenylcarbonyloxy)benzoesäureester;
[4-(4-[6-Acryloyloxyhexyloxy]phenylcarbonyloxy)phenoxy]propyl 2,5-bis(4-[7-acryloyloxyheptyloxy)]phenylcarbonyloxy)benzoesäureester;
[4-(4-[6-Acryloyloxyhexyloxy]phenylcarbonyloxy)phenoxy]ethyl 2,5-bis(4-[8-acryloyloxyoctyloxy)]phenylcarbonyloxy)benzoesäureester;
[4-(4-[6-Acryloyloxyhexyloxy]phenylcarbonyloxy)phenoxy]pentyl 2,5-bis(4-[9-acryloyloxynonyloxy)]phenylcarbonyloxy)benzoesäureester;
[4-(4-[6-Acryloyloxyhexyloxy]phenylcarbonyloxy)phenoxy]hexyl 2,5-bis(4-[10-acryloyloxydecyloxy)]phenylcarbonyloxy)benzoesäureester;
[4-(4-[6-Acryloyloxyhexyloxy]phenylcarbonyloxy)phenoxy]heptyl 2,5-bis(4-[11-acryloyloxyundecyloxy)]phenylcarbonyloxy)benzoesäureester;
[4-(4-[6-Acryloyloxyhexyloxy]phenylcarbonyloxy)phenoxy]octyl 2,5-bis(4-[12-acryloyloxydodecyloxy)]phenylcarbonyloxy)benzoesäureester.

### Beispiel 4

0,3 g 4-[6-Acryloyloxyhexyloxy]benzoesäure, 0,8 g 4-Hydroxybutyl 2,5-bis(4-[11-acryloyloxyundecyloxy)]phenylcarbonyloxy)benzoesäureester, 0,04 g 4-Dimethylaminopyridin und 0,2 g N, N'-Dicyclodicyclohexyldicarbodiimid und 50 ml Dichlormethan werden in analoger Weise zu Beispiel 1 zu 0,5 g 4-(4-[6-Acryloyloxyhexyloxy]phenylcarbonyloxy)butyl 2,5-bis(4-[11-acryloyloxyundecyloxy)]henylcarbonyloxy)benzoesäureester umgesetzt. In analoger Weise können folgende Verbindungen hergestellt werden:
3-(4-[6-Acryloyloxyhexyloxy]phenylcarbonyloxy)propyyl 2,5-bis(4-[11-acryloyloxyundecyloxy)]phenylcarbonyloxy)benzoesäureester;
5-(4-[6-Acryloyloxyhexyloxy]phenylcarbonyloxy)pentyl 2,5-bis(4-[11-acryloyloxyundecyloxy)]phenylcarbonyloxy)benzoesäureester;
6-(4-[6-Acryloyloxyhexyloxy]phenylcarbonyloxy)hexyl 2,5-bis(4-[11-acryloyloxyundecyloxy)]phenylcarbonyloxy)benzoesäureester;
7-(4-[6-Acryloyloxyhexyloxy]phenylcarbonyloxy)heptyl 2,5-bis(4-[11-acryloyloxyundecyloxy)]phenylcarbonyloxy)benzoesäureester;
8-(4-[6-Acryloyloxyhexyloxy]phenylcarbonyloxy)octyl 2,5-bis(4-[11-acryloyloxyundecyloxy)]phenylcarbonyloxy)benzoesäureester;
9-(4-[6-Acryloyloxyhexyloxy]phenylcarbonyloxy)nonyl 2,5-bis(4-[11-acryloyloxyundecyloxy)]phenylcarbonyloxy)benzoesäureester;
10-(4-[6-Acryloyloxyhexyloxy]phenylcarbonyloxy)decyl 2,5-bis(4-[11-acryloyloxyundecyloxy)]phenylcarbonyloxy)benzoesäureester;
11-(4-[6-Acryloyloxyhexyloxy]phenylcarbonyloxy)undecyl 2,5-bis(4-[11-acryloyloxyundecyloxy)]phenylcarbonyloxy)benzoesäureester;
12-(4-[6-Acryloyloxyhexyloxy]phenylcarbonyloxy)dodecyl 2,5-bis(4-[11-acryloyloxyundecyloxy)]phenylcarbonyloxy)benzoesäureester;
3-(4-[6-Acryloyloxyhexyloxy]phenylcarbonyloxy)propyl 2,5-bis(4-[5-acryloyloxypentyloxy)]phenylcarbonyloxy)benzoesäureester;
3-(4-[6-Acryloyloxyhexyloxy]phenylcarbonyloxy)propyl 2,5-bis(4-[6-acryloyloxyhexyloxy)]phenylcarbonyloxy)benzoesäureester;
3-(4-[6-Acryloyloxyhexyloxy]phenylcarbonyloxy)propyl 2,5-bis(4-[7-acryloyloxyheptyloxy)]phenylcarbonyloxy)benzoesäureester;
3-(4-[6-Acryloyloxyhexyloxy]phenylcarbonyloxy)propyl 2,5-bis(4-[8-acryloyloxyoctyloxy)]phenylcarbonyloxy)benzoesäureester;
3-(4-[6-Acryloyloxyhexyloxy]phenylcarbonyloxy)propyl 2,5-bis(4-[9-acryloyloxynonyloxy)]phenylcarbonyloxy)benzoesäureester;
3-(4-[6-Acryloyloxyhexyloxy]phenylcarbonyloxy)propyl 2,5-bis(4-[10-acryloyloxydecyloxy)]phenylcarbonyloxy)benzoesäureester.

### Beispiel 5

Ein Gemisch (C-N, <20^{o}C, N-I, 38^{o}C) aus 80 Gew.% 1,2,4-Tri(4-[6-acryloyloxyhexyloxy]phenylcarbonyloxy)benzol und 20 Gew.% 1 Chlor-2,5-bis(4-[6-Acryloyloxyhexyloxy)]phenylcarbonyloxy)benzol wurde vorgelegt, mit 1 Gew.% eines Photoinitiators (IRGACURE, Ciba Geigy) versetzt, in Anisol gelöst (40 Gew.%) und dann bei 2000 Umdrehungen pro Minute auf eine Glasplatte geschleudert. Die Glasplatte wurde zuvor mit Methacryloyloxyethyl-3-(E)-[4-cyano-4'-biphenyl]acrylat beschichtet und dann mit linear polarisiertem Licht bestrahlt. Dabei wurde eine vorgegebene Struktur mittels einer Maske in die (PPN) Schicht photolithographisch eingeschrieben. Die aufgeschläuderte neue Schicht (auf der PPN Schicht) wurde bei 90^{o}C auf einer Wärmebark getrocknet, dann im Vakuumschank unter Vakuum bei 90^{o}C mit Xenon-Licht berichtet und vernetzt. Die eingeschriebene Originalstruktur blieb erhalten und wurde vom neuen Netzwerk getreu übernommen. Eine klare Doppelbrechung (Δn) war erkennbar. Diese Schicht fungiert als ein strukturierter optischer Retarder.

## Patentansprüche

1. Verbindungen der allgemeinen Formel 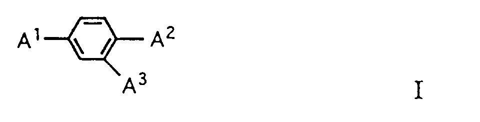 in welcher
A¹, A^{²}, A³ unabhängig voneinander für je einen photochemisch oligomerisierbaren oder polymerisierbaren mesogenen Rest der Formel 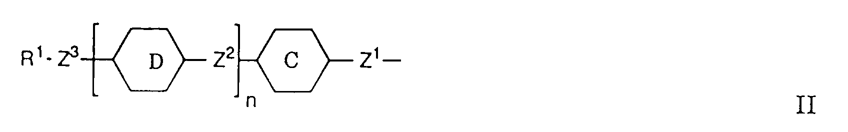
stehen, worin
C und D unabhängig voneinander gegebenenfalls mit Nitro, Acetyl, Halogen, Methyl und/oder Cyano substituiertes 1,4-Phenylen, Pyridin-2,5-diyl, Pyrimidin-2,5-diyl, trans-1,4-Cyclohexylen oder trans-1,3-Dioxan-2,5-diyl;
Z¹ eine Einfachbindung, -CH₂CH₂-, -CH₂O-, -COO-, -OOC-, -(CH₂)₄- oder -(CH₂)₃O- ;
Z² eine Einfachbindung, -CH₂CH₂-, -CH₂O-, -OCH₂-, -COO-, -OOC-, -(CH₂)₄-, -O(CH₂)₃- oder -(CH₂)₃O-;
Z³ -(CY₂)ₘ-, -(CY₂)ₘO-, -(CY₂)ₘCOO-, -(CY₂)ₘOOC, -(Si[(CH₃)₂]O)ₚ-, -OCH₂(Si[(CH₃)₂]O)ₚSi[(CH₃)₂]CH₂O- oder -NHCH₂(Si[(CH₃)₂]O)ₚSi[(CH₃)₂]CH₂NH-;
Y Wasserstoff oder Fluor;
m eine ganze Zahl von 1 bis 16;
p eine ganze Zahl von 1 bis 16;
n 0 oder 1; und
R¹ eine polymerisierbare Gruppe wie Acrylat, Allylether, Allylester, Methacrylat, 2-Chlor-acrylat, 2-Phenylacrylat, Acryloylphenylen, Acrylamid, Methacrylamid, 2-Phenylacrylamid, Epoxy, Itaconsäureester, Vinylether, Vinylester, Styrol-Derivate, Maleinsäure-Derivate, Fumarsäure-Derivate oder eine dimerisierbare Gruppe, wie beispielsweise Chaleon oder ein gegebenenfalls mit Methyl, Methoxy, Cyano und/oder Halogen substituiertes Zimtsäurederivat, bedeuten.

2. Verbindungen gemäss Anspruch 1, dadurch gekennzeichnet, dass zwei der mesogene Reste A¹, A² oder A³ die gleiche Bedeutung haben.

3. Verbindungen gemäss einem der Ansprüche 1 oder 2 mit der Formeln 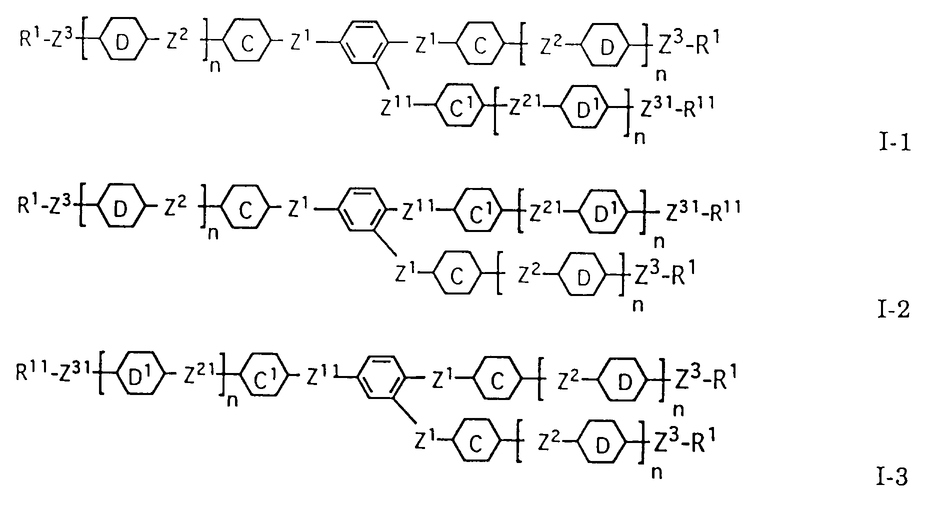 worin
C, C¹, D und D¹ unabhängig voneinander gegebenenfalls mit Nitro, Acetyl, Halogen, Methyl und/oder Cyano substituiertes 1,4-Phenylen, Pyridin-2,5-diyl, Pyrimidin-2,5-diyl, trans-1,4-Cyclohexylen oder trans-1,3-Dioxan-2,5-diyl;
Z¹ und Z¹¹ unabhängig voneinander eine Einfachbindung, -CH₂CH₂-, -CH₂O-, -COO-, -OOC-, -(CH₂)₄- oder -(CH₂)₃O- ;
Z² und Z²¹ unabhängig voneinander eine Einfachbindung, -CH₂CH₂-, -CH₂O-, -OCH₂-, -COO-, -OOC-, -(CH₂)₄-, -O(CH₂)₃- oder -(CH₂)₃O-;
Z³ und Z³¹ unabhängig voneinander -(CY₂)ₘ-, -(CY₂)ₘO-, -(CY₂)ₘCOO-, -(CY₂)ₘOOC, -(Si[(CH₃)₂]O)ₚ-, -OCH₂(Si[(CH₃)₂]O)ₚSi[(CH₃)₂]CH₂O- oder -NHCH₂(Si[(CH₃)₂]O)ₚSi[(CH₃)₂]CH₂NH-;
Y Wasserstoff oder Fluor;
m eine ganze Zahl von 1 bis 16;
p eine ganze Zahl von 1 bis 16;
n 0 oder 1; und
R¹ und R¹¹ unabhängig voneinander eine polymerisierbare Gruppe wie Acrylat, Allylether, Allylester, Methacrylat, 2-Chlor-acrylat, 2-Phenylacrylat, Acryloylphenylen, Acrylamid, Methacrylamid, 2-Phenylacrylamid, Epoxy, Itaconsäureester, Vinylether, Vinylester, Styrol-Derivate, Maleinsäure-Derivate, Fumarsäure- Derivate oder eine dimerisierbare Gruppe, wie beispielsweise Chalcon oder ein gegebenenfalls mit Methyl, Methoxy, Cyano und/oder Halogen substituiertes Zimtsäurederivat, bedeuten.

4. Verbindungen gemäss Anspruch 1, mit der Formel 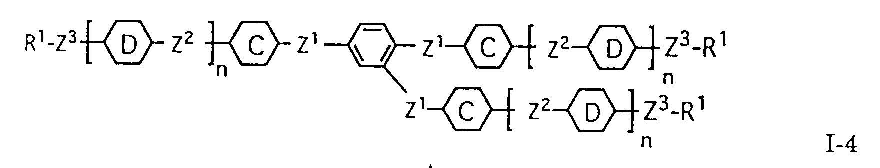 worin die Symbole die im Anspruch 1 angegebenen Bedeutungen haben.

5. Verbindungen gemäss einem der Ansprüche 1 bis 4, worin Z¹ bzw. Z¹¹ -CH₂O-, -COO- oder -OOC-; Z² bzw. Z²¹ eine Einfachbindung, -CH₂CH₂, -CH₂O-, -OCH₂-, -COO- oder -OOC-; und Y Wasserstoff bedeuten.

6. Verbindungen gemäss Anspruch 5, worin R¹ bzw. R¹¹ Acrylat, Methacrylat, Vinylether oder Epoxy bedeuten.

7. Verbindungen gemäss Anspruch 1, dadurch gekennzeichnet, dass die mesogenen Reste A¹, A² und A³ einen Rest der Formel 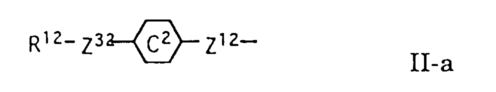 bedeutet, worin
R¹² Acrylat, Methacrylat oder Epoxy;
Z³² -(CH₂)ₜ-, -(CH₂)ₜO-, -(CH₂)ₜCOO- oder -(CH₂)ₜOOC-;
t eine ganze Zahl von 3 bis 12;
C² 1,4-Phenylen, 2-Fluor-1,4-phenylen, 3-Fluor-1,4-phenylen; und
Z¹² -CH₂O-, -COO- oder -OOC- bedeutet.

8. Verbindungen gemäss einem der Ansprüche 1 und 4 bis 7 der Formel 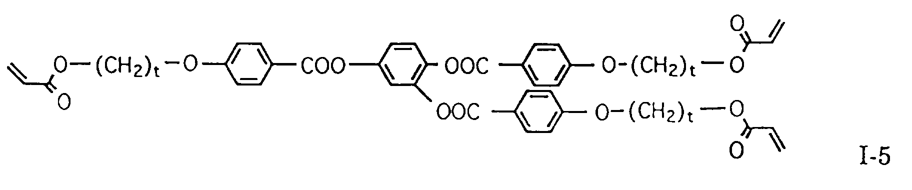 worin t eine ganze Zahl von 3 bis 12 bedeutet.

9. Polymerisierbare, flüssigkristalline Gemische bestehend aus mindestens 2 Komponenten, wovon mindestens eine Komponente eine Verbindung der in Anspruch 1 definierten Formel I ist.

10. Polymerisierbare, flüssigkristalline Gemische gemäss Anspruch 9, dadurch gekennzeichnet, dass sie neben einer oder mehreren Verbindungen der Formel I, eine oder mehrere Verbindungen aus der Gruppe der Verbindungen der Formeln 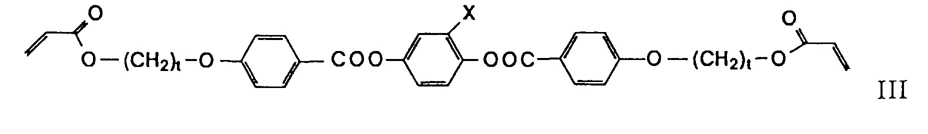 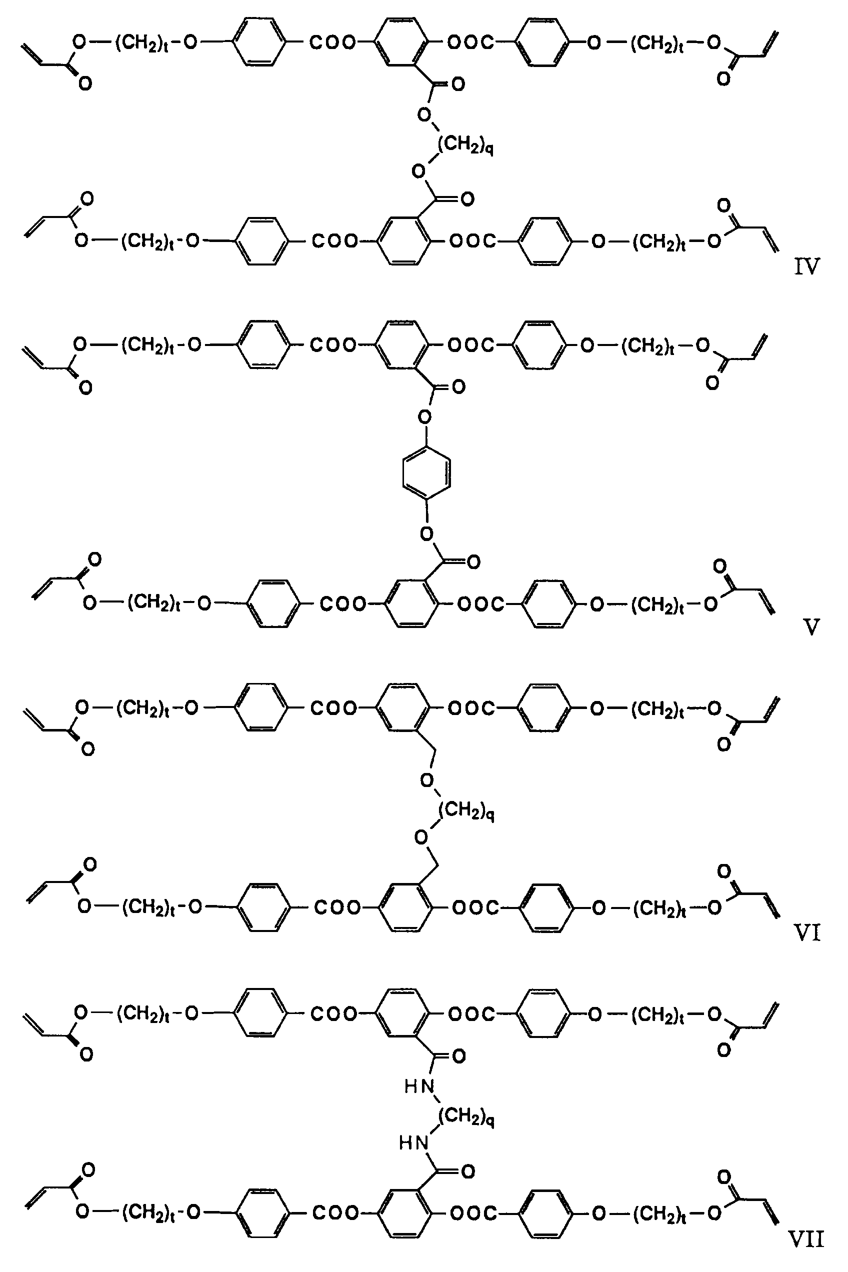 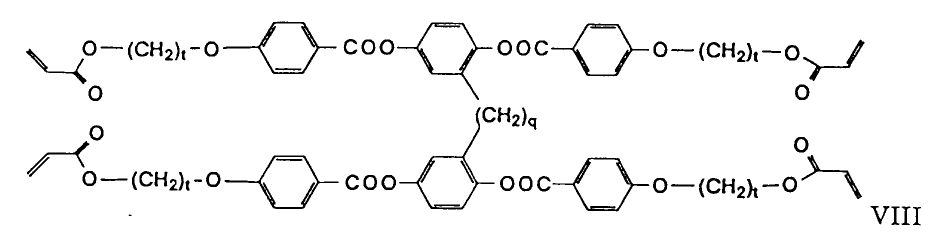 worin
t eine ganze Zahl von 3 bis 12;
q eine ganze Zahl von 2 bis 12; und
X Wasserstoff, niederes Alkyl, Fluor oder Chlor bedeuten.

11. Verwendung von Verbindungen gemäss einem der Ansprüche 1 bis 8 in ihrem vernetzten Zustand für optische Bauelemente.

12. Verwendung von photochemisch oligomerisierbaren oder polymerisierbaren flüssigkristallinen Gemischen gemäss einem der Ansprüche 9 oder 10 in ihrem vernetzten Zustand für optische oder elektronische Bauelemente.

## Claims

1. Compounds of the general formula 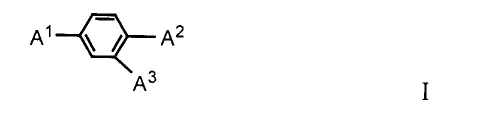 wherein
A¹, A², A³ each independently signify photochemically oligomerisable or polymerisable mesogenic residues of the formula 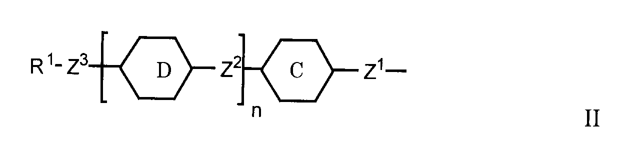
wherein
C and D each independently signify 1,4-phenylene optionally substituted by nitro, acetyl, halogen, methyl and/or cyano, pyridine-2,5-diyl, pyrimidine-2,5-diyl, trans-1,4-cyclohexylene or trans-1,3-dioxane-2,5-diyl;
Z¹ signifies a single bond, -CH₂CH₂-, -CH₂O-, -COO-, -OOC-, -(CH₂)₄- or -(CH₂)₃O- ;
Z² signifies a single bond, -CH₂CH₂-, -CH₂O-, -OCH₂-, -COO-, -OOC-, -(CH₂)₄-, -O(CH₂)₃- or -(CH₂)₃O-;
Z³ signifies -(CY₂)ₘ-, -(CY₂)ₘO-, -(CY₂)ₘCOO-, -(CY₂)ₘOOC, -(Si[(CH₃)₂]O)ₚ-, -OCH₂(Si[(CH₃)₂]O)ₚSi[(CH₃)₂]CH₂O- or -NHCH₂(Si[(CH₃)₂]O)ₚSi[(CH₃)₂]CH₂NH-;
Y signifies hydrogen or fluorine;
m signifies a whole number of 1 to 16;
p signifies a whole number of 1 to 16;
n signifies 0 or 1; and
R¹ signifies a polymerisable group such as acrylate, allyl ether, allyl ester, methacrylate, 2-chloroacrylate, 2-phenylacrylate, acryloylphenylene, acrylamide, methacrylamide, 2-phenylacrylamide, epoxy, itaconic acid ester, vinyl ether, vinyl ester, styrene derivative, maleic acid derivative, fumaric acid derivative or a dimerisable group such as, for example, chalcone or a cinnamic acid derivative optionally substituted by methyl, methoxy, cyano and/or halogen.

2. Compounds according to claim 1, characterised in that two of the mesogenic residues A¹, A² and A³ have the same significance.

3. Compounds according to any one of claims 1 and 2 of the formulae 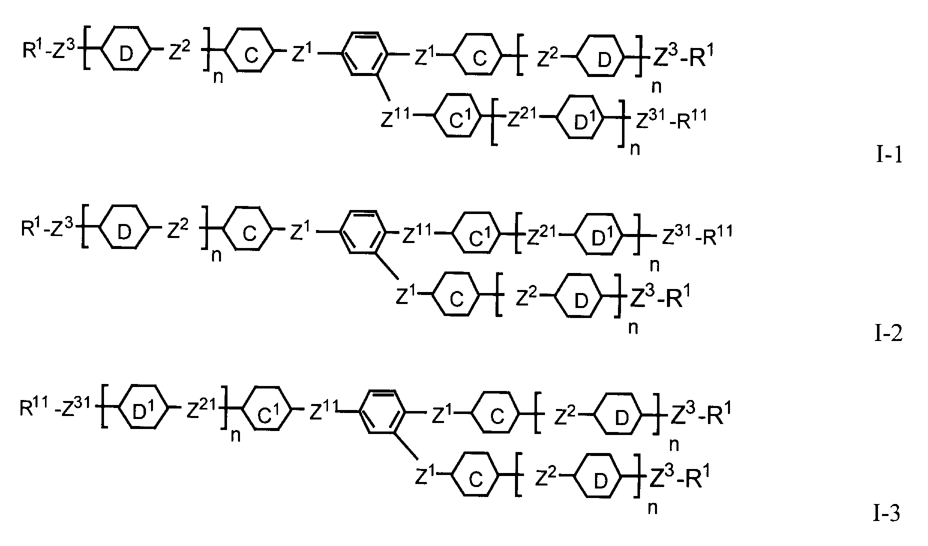 wherein
C, C¹, D and D¹ each independently signify 1,4-phenylene optionally substituted by nitro, acetyl, halogen, methyl and/or cyano, pyridine-2,5-diyl, pyrimidine-2,5-diyl, trans-1,4-cyclohexylene or trans-1,3-dioxane-2,5-diyl;
Z¹ and Z¹¹ each independently signify a single bond, -CH₂CH₂-, -CH₂O-, -COO-, -OOC-, -(CH₂)₄- or -(CH₂)₃O-;
Z² and Z²¹ each independently signify a single bond, -CH₂CH₂-, -CH₂O-, -OCH₂-, -COO-, -OOC-, -(CH₂)₄-, -O(CH₂)₃- or -(CH₂)₃O-;
Z³ and Z³¹ each independently signify -(CY₂)ₘ-, -(CY₂)ₘO-, -(CY₂)ₘCOO-, -(CY₂)ₘOOC, -(Si[(CH₃)₂]O)ₚ-, -OCH₂(Si[(CH₃)₂]O)ₚSi[(CH₃)₂]CH₂O- or -NHCH₂(Si[(CH₃)₂]O)ₚSi[(CH₃)₂]CH₂NH-;
Y signifies hydrogen or fluorine;
m signifies a whole number of 1 to 16;
p signifies a whole number of 1 to 16;
n signifies 0 or 1; and R¹ and R¹¹ each independently signify a polymerisable group such as
acrylate, allyl ether, allyl ester, methacrylate, 2-chloroacrylate, 2-phenylacrylate, acryloylphenylene, acrylamide, methacrylamide, 2-phenylacrylamide, epoxy, itaconic acid ester, vinyl ether, vinyl ester, styrene derivative, maleic acid derivative, fumaric acid derivative or a dimerisable group such as, for example, chalcone or a cinnamic acid derivative optionally substituted by methyl, methoxy, cyano and/or halogen.

4. Compounds according to claim 1 of the formula 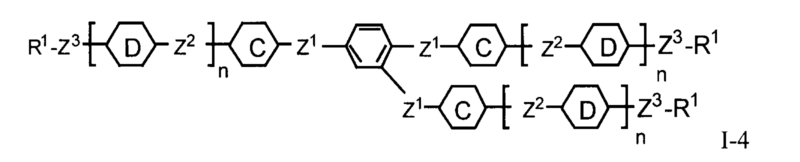 wherein the symbols have the significances given in claim 1.

5. Compounds according to any one of claims 1 to 4, wherein Z¹ and/or, as the case may be, Z¹¹ signify -CH₂O-, -COO- or -OOC-; Z² and/or, as the case may be, Z²¹ signify a single bond, -CH₂CH₂, -CH₂O-, -OCH₂-, -COO- or -OOC-; and Y signifies hydrogen.

6. Compounds according to claim 5, wherein R¹ and/or, as the case may be, R¹¹ signify acrylate, methacrylate, vinyl ether or epoxy.

7. Compounds according to claim 1, characterised in that the mesogenic residues A¹, A² and A³ signify a residue of the formula 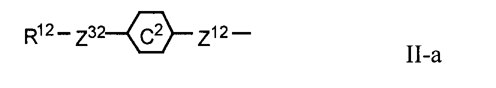 wherein
R¹² signifies acrylate, methacrylate or epoxy;
Z³² signifies -(CH₂)ₜ, -(CH₂)ₜO-, -(CH₂)ₜCOO- or -(CH₂)ₜOOC-;
t signifies a whole number of 3 to 12;
C² signifies 1,4-phenylene, 2-fluoro-1,4-phenylene or 3-fluoro-1,4-phenylene; and
Z¹² signifies -CH₂O-, -COO- or -OOC-.

8. Compounds according to any one of claims 1 and 4 to 7 of the formula 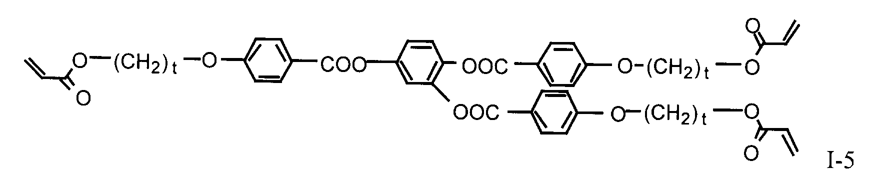 wherein t signifies a whole number of 3 to 12.

9. Polymerisable, liquid crystalline mixtures consisting of at least two components, of which at least one component is a compound of formula I defined in claim 1.

10. Polymerisable, liquid crystalline mixtures according to claim 9, characterised in that they comprise, in addition to one or more compounds of formula I, one or more compounds from the group of compounds of the formulae 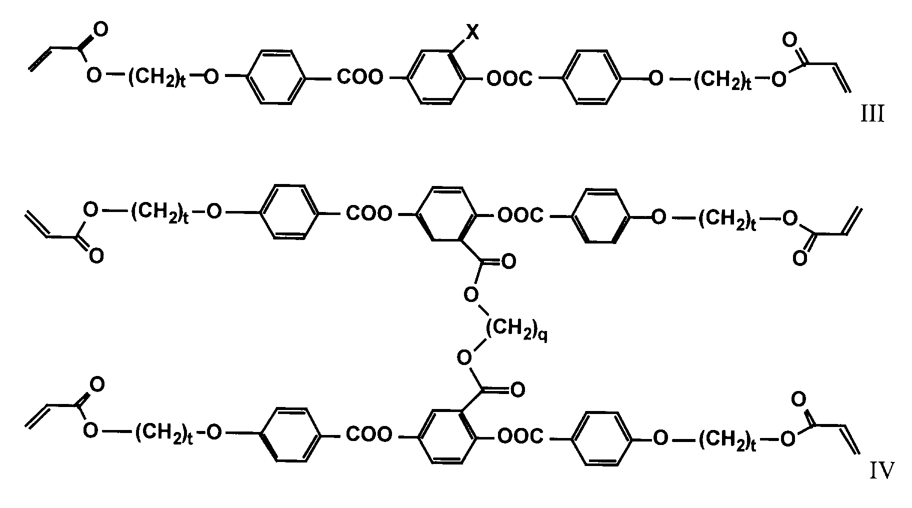 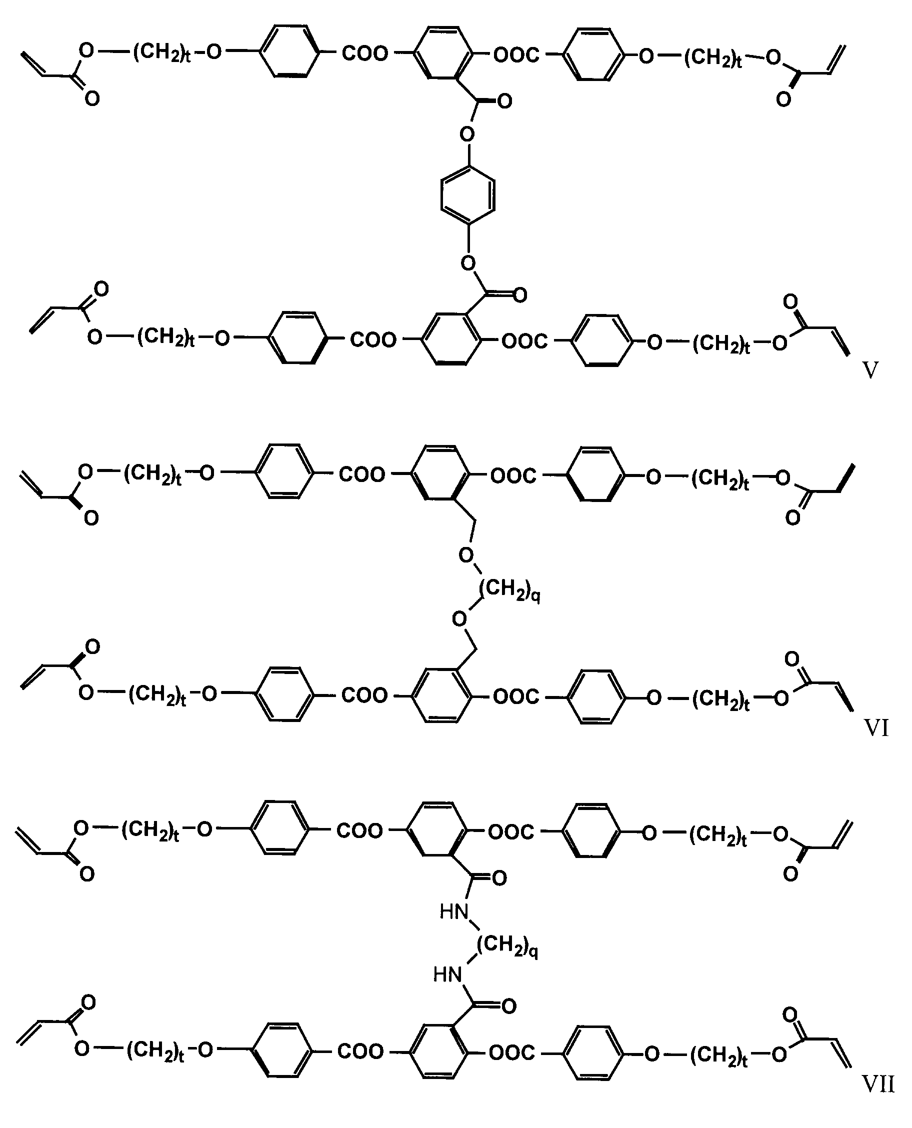 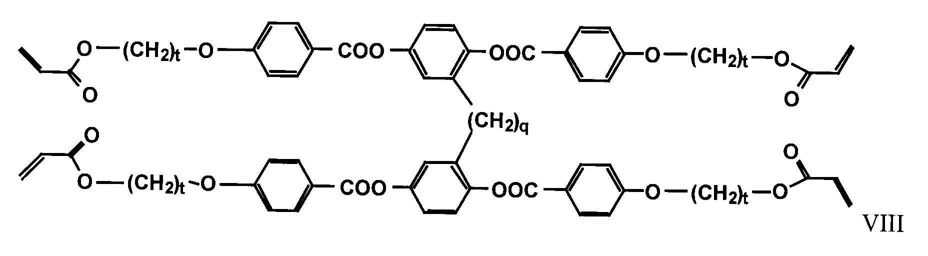 wherein t signifies a whole number of 3 to 12; q signifies a whole number of 2 to 12; and X signifies hydrogen, lower alkyl, fluorine or chlorine.

11. The use of compounds according to any one of claims 1 to 8 in their cross-linked state for optical components.

12. The use of photochemically oligomerisable or polymerisable, liquid crystalline mixtures according to claim 9 or 10 in their cross-linked state for optical or electronic components.

## Revendications

1. Composés de formule générale 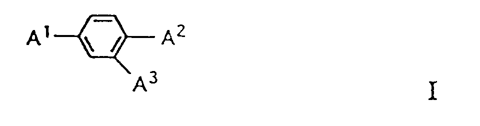 dans laquelle
A¹, A², A³ représentent indépendamment les uns des autres un reste mésogène polymérisable ou oligomérisable photochimiquement de formule 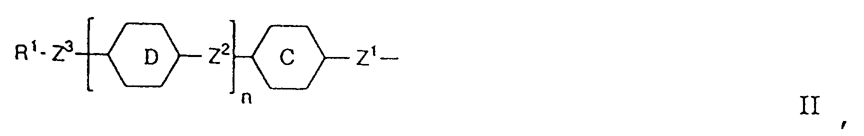
dans laquelle
C et D représentent indépendamment l'un de l'autre un groupe 1,4-phénylène éventuellement substitué par un groupe nitro, acétyle, un atome d'halogène, un groupe méthyle et/ou cyano, pyridin-2,5-diyle, pyrimidin-2,5-diyle, trans-1,4-cyclohexylène ou trans-1,3-dioxan-2,5-diyle;
Z¹ représente une liaison simple, -CH₂CH₂-, -CH₂O-, -COO-, -OOC-, -(CH₂)₄- ou -(CH₂)₃O-;
Z² représente une liaison simple, -CH₂CH₂-, -CH₂O-, -OCH₂-, -COO-, -OOC-, -(CH₂)₄-, -O(CH₂)₃- ou -(CH₂)₃O-;
Z³ représente -(CY₂)ₘ-, -(CY₂)ₘO-, -(CY₂)ₘCOO-, -OCH₂(Si[(CH₃)₂]O)ₚSi[(CH₃)₂]CH₂O-, ou -NHCH₂(Si[(CH₃)₂]O)ₚSi[(CH₃)₂]CH₂NH-;
Y représente un atome d'hydrogène ou de fluor;
m représente un nombre entier de 1 à 16;
p représente un nombre entrer de 1 à 16;
n représente 0 ou 1; et
R¹ représente un groupe polymérisable tel qu'un groupe acrylate, éther allylique, ester allylique, méthacrylate, 2-chloro-acrylate, 2-phénylacrylate, acryloylphénylène, acrylamide, méthacrylamide, 2-phénylacrylamide, époxy, ester d'acide itaconique, éther vinylique, ester de vinyle, les dérivés du styrène, les dérivés de l'acide maléique, les dérivés de l'acide fumarique ou un groupe dimérisable comme par exemple une chalcone ou un dérivé de l'acide cinnamique éventuellement substitué par un groupe méthyle, méthoxy, cyano et/ou un atome d'halogène.

2. Composés selon la revendication 1, caractérisés en ce que, deux des restes mésogènes A¹, A² ou A³ ont une signification identique.

3. Composés selon l'une des revendications 1 ou 2, ayant les formules 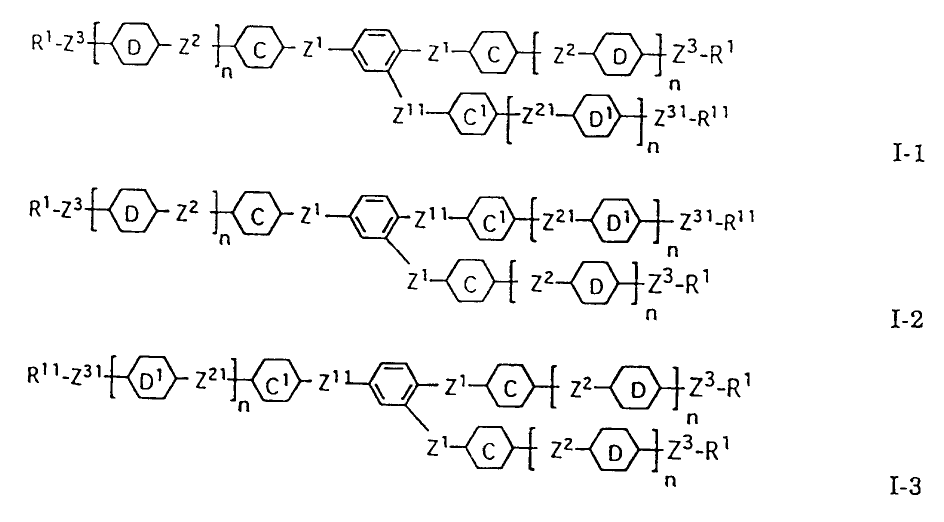 dans lesquelles
C, C¹, D et D¹ représentent indépendamment les uns des autres un groupe 1,4-phénylène éventuellement substitué par un groupe nitro, acétyle, un atome d'halogène, un groupe méthyle et/ou cyano, pyridin-2,5-diyle, pyrimidin-2,5-diyle, trans-1,4-cyclohexylène ou trans-1,3-dioxan-2,5-diyle;
Z¹ et Z¹¹ représentent indépendamment l'un de l'autre une liaison simple, -CH₂CH₂ -, -CH₂O-, -COO-, -OOC-, -(CH₂)₄-, -O(CH₂)₃- ou -(CH₂)₃O-;
Z² et Z²¹ représentent indépendamment l'un de l'autre une liaison simple, -CH₂CH₂-, -CH₂O-, -OCH₂-, -COO-, -OOC-, -(CH₂)₄-, ou -(CH₂)₃O-;
Z³ et Z³¹ représentent indépendamment l'un de l'autre -(CY₂)ₘ-, -(CY₂)ₘO-, -(CY₂)ₘCOO-, -(CY₂)ₘOOC-, -(Si[(CH₃)₂]O)ₚ-, -OCH₂(Si[(CH₃)₂]O)ₚSi[(CH₃)₂]CH₂O-, ou -NHCH₂(Si[(CH₃)₂]O)ₚSi[(CH₃)₂]CH₂NH-;
Y représente un atome d'hydrogène ou de fluor;
m représente un nombre entier de 1 à 16;
p représente un nombre entier de 1 à 16;
n représente 0 ou 1; et
R¹ et R¹¹ représentent indépendamment l'un de l'autre un groupe polymérisable tel qu'un groupe acrylate, éther allylique, ester allylique, méthacrylate, 2-chloro-acrylate, 2-phénylacrylate, acryloyl-phénylène, acrylamide, méthacrylamide, 2-phénylacrylamide, époxy, ester d'acide itaconique, éther vinylique, ester de vinyle, les dérivés du styrène, les dérivés de l'acide maléique, les dérivés de l'acide fumarique ou un groupe dimérisable comme par exemple une chalcone ou un dérivé de l'acide cinnamique éventuellement substitué par un groupe méthyle, méthoxy, cyano et/ou un atome d'halogène.

4. Composés selon la revendication 1, ayant la formule 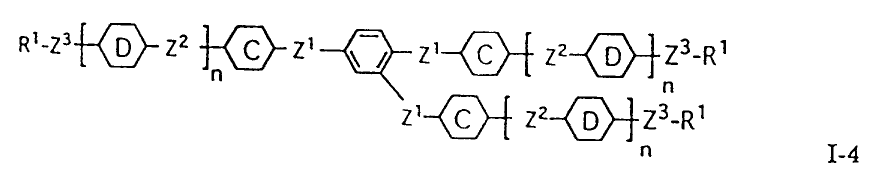 dans laquelle les symboles ont les significations données à la revendication 1.

5. Composés selon l'une des revendications 1 à 4, dans lesquels Z¹ ou Z¹¹ représente -CH₂O-, -COO- ou -OOC-; Z² ou Z²¹ représente une liaison simple, -CH₂CH₂-, -CH₂O-, -OCH₂-, -COO- ou -OOC-; et Y représente un atome d'hydrogène.

6. Composés selon la revendication 5, dans lesquels R¹ ou R¹¹ représente un groupe acrylate, méthacrylate, éther vinylique ou époxy.

7. Composés selon la revendication 1, caractérisés en ce que les restes mésogènes A¹, A² et A³ représentent un groupe de formule 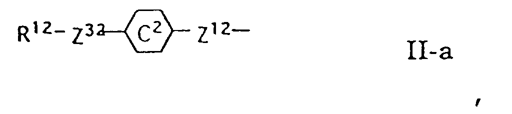 dans laquelle
R¹² représente un groupe acrylate, méthacrylate ou époxy;
Z³² représente -(CH₂)ₜ-, -(CH₂)ₜO-, -(CH₂)ₜCOO- ou -(CH₂)ₜOOC-;
t est un nombre entier de 3 à 12;
C² représente un groupe 1,4-phénylène, 2-fluoro-1,4-phénylène, 3-fluoro-1,4-phénylène; et
Z¹² représente -(CH₂)O-, -COO- ou -OOC-.

8. Composés selon l'une des revendications 1, 2 et 4 à 7, de formule 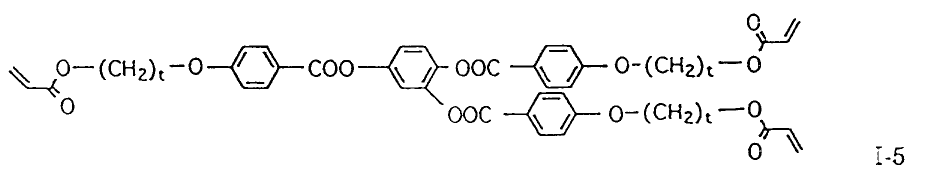 dans laquelle t représente un nombre entier de 3 à 12.

9. Mélange polymérisable, a cristaux liquides constitué d'au moins deux composants, parmi lesquels au moins un composant est un composé de formule I telle que définie à la revendication 1.

10. Mélange polymérisable, à cristaux liquides, selon la revendication 9, caractérisé en ce qu'il contient en plus d'un ou de plusieurs composé(s) de formule I, un ou plusieurs composés choisi(s) parmi les composés des formules 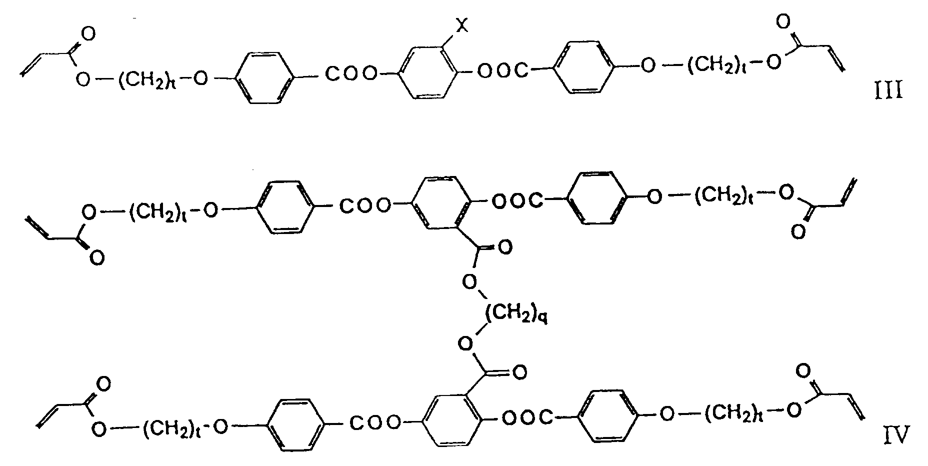 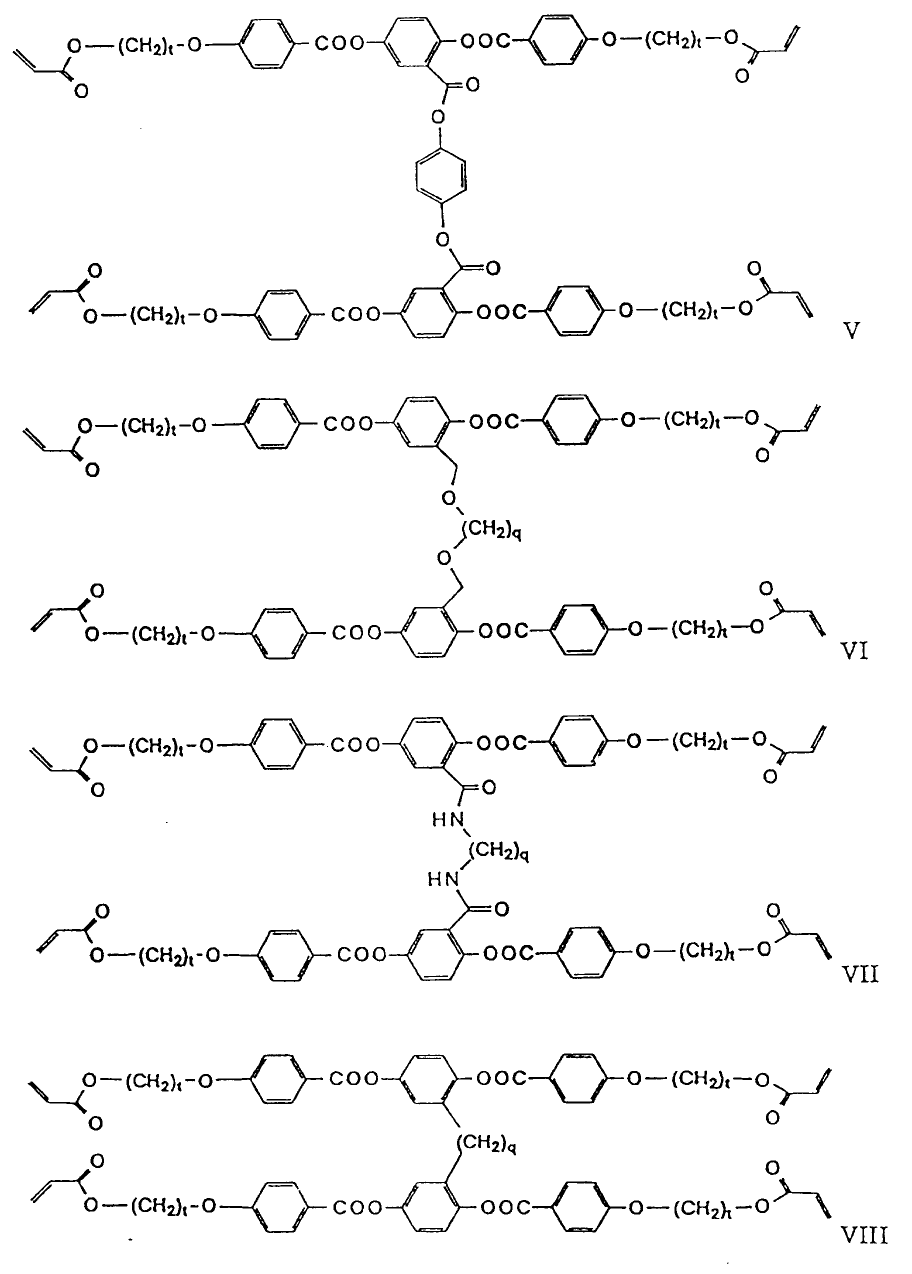 dans lesquelles
t représente un nombre entier de 3 à 12;
q représente un nombre entier de 2 à 12; et
X représente un atome d'hydrogène, un groupe alkyle inférieur, un atome de fluor ou de chlore.

11. Utilisation des composés selon l'une des revendications 1 à 8 dans leur état réticulé pour des composants optiques.

12. Utilisation des compositions à cristaux liquides polymérisables ou oligomérisables photochimiquement selon l'une des revendications 9 ou 10 dans leur état réticulé pour des composants optiques ou électroniques.
